**Europäisches Patentamt**

**European Patent Office**

**Office européen des brevets**

(11) Veröffentlichungsnummer: **0 146 490**
**A1**

## (12) EUROPÄISCHE PATENTANMELDUNG

(21) Anmeldenummer: **84730096.9**

(22) Anmeldetag: **12.09.84**

(51) Int. Cl.⁴: **A 61 F 5/14**

(30) Priorität: **12.09.83 DE 8326464 U**

(43) Veröffentlichungstag der Anmeldung: **26.06.85**
**Patentblatt 85/26**

(84) Benannte Vertragsstaaten: **AT BE CH DE FR GB IT LI NL SE**

(71) Anmelder: **Dittmers, Egon, Schillersstrasse 26, D-2057 Reinbek (DE)**

(72) Erfinder: **Dittmers, Egon, Schillersstrasse 26, D-2057 Reinbek (DE)**

(74) Vertreter: **Patentanwälte Wenzel & Kalkoff, Grubes Allee 26 Postfach 730466, D-2000 Hamburg 73 (DE)**

(54) **Orthopädische Richthilfe für Zehen.**

(57) Eine orthopädische Richthilfe für Zehen des menschlichen Fusses umfasst eine den Zeh untergreifende Auflagefläche (23) sowie seitliche Anlagen (32) für den Zeh. Um eine möglichst individuell anpassbare Ausrichtung von verkrümmten Zehen zu ermöglichen, besteht die Richthilfe aus einem prinzipiell festen und formbeständigen, im Querschnitt im wesentlichen U-förmigen, sich in Richtung der Zehenachse erstreckenden schienenartigen Formstück (1), dessen U-Steg (21) die Auflagefläche (23) und dessen U-Schenkel (3) die seitlichen Anlagen (32) bilden.

0146490

- 1 -                                    11.09.1984

Egon Dittmers, Schillerstraße 26, 2057 Reinbek

Orthopäische Richthilfe für Zehen

Die Erfindung betrifft eine orthopädische Richthilfe für Zehen des menschlichen Fußes, umfassend eine den Zeh untergreifende Auflagefläche sowie seitliche Anlagen für den Zeh.

Es ist eine bekannte Folge des in der heutigen Zivilisation immer enger werdenden Schuhwerks, daß Zehen Verformungen erleiden, die zu starken Verkümmerungen der Sehnen mit erheblichen Schmerzen führen. Dieses Symptom, ausgelöst durch in der Regel zu kleines bzw. zu enges Schuh- und/oder Strumpfwerk, ist u.a. *als verkrümmter Zeh, seitlich verkrümmt oder* als Hammerzeh bekannt und mit erheblichen Schmerzen bis zu starken Gehbehinderungen hin verbunden.

Um diesen Beschwernissen abzuhelfen, sie zumindest aber zu lindern, sind Zehen-Richter bekannt geworden, die eine unter dem Fußballen liegende weiche Auftrittplatte aufweisen, an der Gummibandschlaufen zum Umfassen mindestens eines Zehs, in der Regel aber zweier benachbarter Zehen angeordnet sind. Diese Schlaufen sind aufgrund ihrer Materialbeschaffenheit und ihrer Anordnung größenverstellbar und umschließen den Zeh auf etwa 1 cm Breite sehr eng, was leicht zu einem Abschnüren der gleichmäßigen Durchblutung des Zehs führen kann. Außerdem sind die Schlaufen nicht geeignet, dem Zeh einen größeren Halt gegen seitliches Ausweichen zu geben. Vielmehr ist es damit nach wie vor möglich, daß der Zeh sich seitlich verdrehen kann, so daß zwar in manchen Fällen eine Entkrümmung des Zehs in einer Richtung stattfindet, insbesondere eine Streckung, d.h. Entkrümmung von Hammerzehen, während andererseits wieder eine Verformung in anderer Richtung befürchtet werden muß.

Der Erfindung liegt die Aufgabe zugrunde, demgegenüber eine möglichst einfache, individuell anpaßbare orthopädische

~~Aus~~Richthilfe zu schaffen.

Diese Aufgabe wird erfindungsgemäß dadurch gelöst, daß die
Richthilfe aus einem prinzipiell festen und formbeständigen,
im Querschnitt im wesentlichen U-förmigen, sich in Richtung
der Zehenachse erstreckenden schienenartigen Formstück besteht, dessen U-Steg die Auflagefläche und dessen U-Schenkel
die seitlichen Anlagen bilden. Damit erhält man eine Möglichkeit, mittels des Formstücks Zehen einzeln gerade auch
im Schuh unter individueller Anpassung an die Zehenform
so eng zu umgeben, daß der Zeh nicht mehr ausweichen kann,
während zum anderen ein Untergreifen des gesamten Fußballens
durch die Auflageplatte nicht notwendig ist. Die Erfindung
schafft einen Schlitten bzw. eine Schiene, mit der Zehen-
Verformungen sowohl in vertikaler wie auch in horizontaler
Ebene allmählich zurückgebildet werden können. Diese Schlitten oder Schienen sind relativ einfach herstellbar und vom
Materialaufwand her kostengünstig. Sie umgeben praktisch
*von drei Seiten her*
nur den Zeh in seiner gesamten Erstreckung, so daß sie
bequem in einem Schuh getragen werden können, ohne die
Gehfähigkeit maßgeblich zu behindern.

Um eine besonders günstige Anpassung an die Grundform
menschlicher Zehen zu erreichen, können die U-Schenkel
vorzugsweise      auf einem Teil ihrer Längserstreckung
eine schwache Wölbung nach außen aufweisen, damit das den
breitesten Teil eines Zehs bildende Zehengelenk umfaßt werden kann und so erreicht wird, daß die Richthilfe am Zeh
anliegt, also sich dessen Kontur anpaßt, und nicht vom Zeh
herunterrutschen kann.

Nach einer anderen bevorzugten Ausbildungsform der Erfindung können die U-Schenkel an einem, dem hinteren Ende des
Formstücks mit gegenüber dem Ende der Auflagefläche zurückspringenden Lappen ausgebildet sein. Diese Lappen sind insbesondere dazu geeignet, den hinteren Teil des Zehs eng zu

0146490

umschließen. In Anpassung an die unterschiedlichen Zehenformen des menschlichen Fußes können die Lappen leicht
gewölbt sein. Außerdem kann speziell für Formstücke für die
große Zehe einer der Lappen als längliche Lasche ausgebildet sein, die seitlich gegen die Zehenwurzel anliegt, um
unter Wirkung des von außen andrückenden Schuhs eine Richtkomponente auf den von dem Formstück umgriffenen Zeh auszuüben. Auch können *vorteilhaft* die beiden Lappen unterschiedlich lang
sein, um den orthopädischen Gegebenheiten einer vom großen
Zeh nach außen bogenförmig verlaufenden Anordnung der Zehenansätze Rechnung zu tragen.

Weiterhin können an einem, dem vorderen Ende des Formstücks
die Schenkelränder - vorteilhaft leicht - nach innen gebogen
sein. Es ergibt sich damit eine enge Anlage der Richthilfe
in dem Bereich der Zehenspitze, so daß der Zeh nicht durch
die vordere Öffnung des Formstücks hindurchrutschen kann.
Bei einer weiteren Ausführungsform der Erfindung kann im
vorderen Bereich der Auflagefläche, also des U-Steges des
Formstücks, eine plattenartige ebene Vertiefung zur Auflage
der Zehenbeere vorgesehen sein. Dadurch erhält der Zeh im
Bereich seiner natürlichen Kontaktfläche zum Boden, also
der Beere, eine besonders breite Auflage, wodurch seine
Haftung an dem Formstück erhöht wird. Nach hinten kann sich
die Auflagefläche von dieser Vertiefung unter gewisser Anpassung an die Unterseite der Zehe leicht gewölbt ansteigend erstrecken.

Im Rahmen einer günstigen Anpassung an die üblichen Konturen eines menschlichen Zehs und dessen orthopädischen Aufbau können die Oberkanten der U-Schenkel von den Lappen her
zum gegenüberliegenden, vorderen Ende hin leicht abfallen,
so daß die U-Schenkel den Zeh in den flacheren vorderen Bereichen nicht unnötig überragen und somit nach oben kein
Hindernis für Schuh- oder Strumpfwerk darstellen können.

Damit man bei angelegtem Formstück bequem in einen Strumpf, Schuh oder eine Sandale gleiten kann, sollte der U-Steg mindestens im Bereich seines vorderen Abschlußrandes eine leichte Aufwölbung aufweisen. Diese Aufwölbung bildet dann das freie, vom Fuß wegweisende Ende der Richthilfe und verhindert ein Festhaken an bzw. Hinterfangen von anderen Gegenständen.

Für das Richten starker vertikaler Verformungen des Zehs, z.B. eines sog. Hammerzehs, kann zwischen den U-Schenkeln eine sich im wesentlichen parallel zu dem U-Steg nur über einen Teil der Länge des Formstücks erstreckende bügelartige Verbindung anbringbar sein, mit der das Zehengelenk langsam wieder heruntergedrückt und der Zeh gestreckt werden kann. Dabei verhindert das U-Profil des Formstücks ein seitliches Ausweichen des Zehs.

Die Richthilfe kann aus einem metallischen Material wie Aluminium, rostfreiem Stahl od.dgl., aber *besonders* auch aus ~~entspre-~~ ~~chen~~dem nichtmetallischem Material wie Kunststoff bestehen. Bei der Materialwahl kommt es darauf an, daß eine relativ leichte Verformbarkeit mit Hilfe von Werkzeugen zum Zwecke der Anpassung an die individuellen Zehenverhältnisse gegeben ist, während andererseits beim Tragen natürlich das Formstück steif genug sein muß, um seine Grundaufgabe, nämlich das Ausrichten des Zehs, zu erfüllen. Metallisches Material kann vorzugsweise gebogen oder tiefgezogen werden, während bei der Herstellung aus Kunststoff die Herstellung durch Spritzen, Gießen oder Ziehen unter Vakuum als vorteilhafte Herstellungsverfahren in Betracht kommen.

Um bei metallischen Materialien eine genügende Oberflächenglätte und ein ästhetisches Aussehen zu schaffen, kann die Richthilfe vorteilhaft mit einem durch Sintern oder Tauchen erzeugten Farbüberzug aus körperfreundlichem Material versehen sein.

0146490

Zweckmäßigerweise kann das Formstück an seinen Innenseiten mit Polsterungen versehen sein, um so den Schlitten ggf. den jeweiligen Umständen entsprechend zu formen und diesen anzupassen und die Bequemlichkeit beim Tragen, die natürlich ein vorrangiges Gebot ist, zu gewährleisten sowie ein Scheuern zu vermeiden.

Weitere Möglichkeiten, Zweckmäßigkeiten und Vorteile der Erfindung gehen aus der folgenden Beschreibung der in der schematischen Zeichnung dargestellten Ausführungsbeispiele hervor. In der Zeichnung zeigt

Fig. 1   in Ansicht von schräg oben einen Rohling für eine erfindungsgemäße Richthilfe,

Fig. 2   eine ausgeformte Richthilfe in axonometrischer Darstellung und

Fig. 3   eine Seitenansicht einer Richthilfe in abgewandelter Ausführung.

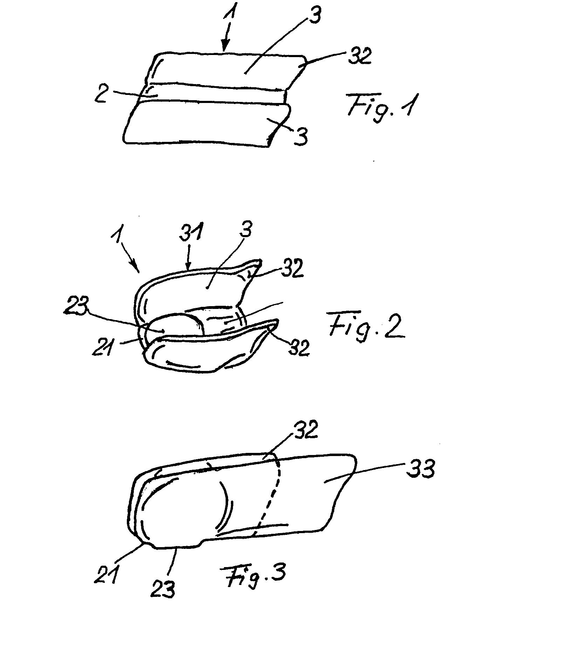

Wie aus der Rohlings-Darstellung der Fig. 1 erkennbar, umfaßt die Richthilfe ein U-förmiges Form- bzw. Schienenstück 1, das im wesentlichen aus einem U-Steg 2 und zwei U-Schenkeln 3 besteht. Ein solches Formstück kann aus Nirosta-Stahl oder Aluminium, aber ebensogut aus Kunstharz bestehen und ist im Prinzip starr, sollte aber eine geringe Restelastizität aufweisen. Ein solcher Rohling kann durch Spanabheben an die gewünschte individuelle Zehenform angepaßt werden.

Die U-Schenkel 3 sind, wie man aus Fig. 2 erkennt, in einem Teilbereich leicht nach außen gewölbt, so daß hier ein günstiges Anpassen an den breiteren Gelenkbereich eines Zehs möglich ist, während sie an ihrem hinteren Ende Lappen 32 tragen, von denen einer, wie aus Fig. 3 erkennbar, länger als der andere ausgebildet sein kann. Der Lappen bildet hier eine Lasche 33, die für den speziellen Einsatz der Richthilfe am großen (rechten) Zeh geeignet ist und zur Anlage gegen die Außenseite des Wurzelbereiches des Zehs dient, um eine genügende Ausrichtung der Richthilfe beim Tragen eines Schuhs zu ermöglichen. Bei für die übrigen vier Zehen eines Fußen geeigneten Richthilfen sind die Lappen 32 nur geringfügig unterschiedlich lang, wodurch eine Anpassung an die orthopädischen Gegebenheiten (bogenförmiger Verlauf der Zehenwurzeln, unterschiedlich lange Zehen von innen nach außen) möglich ist. Der Rand des U-Steges an dem vorderen Ende des Formstücks ist mit einer leichten Aufwölbung 21 versehen. Innerhalb dieser Aufwölbung ist im vorderen Teil des U-Steges 2 eine vertiefte Auflagefläche 23 zur Aufnahme der Zehenbeere, d.h. der vorderen natürlichen Auflagefläche des Zehs, angeordnet.

Patentansprüche:

1. Orthopädische Richthilfe für Zehen des menschlichen
   Fußes, umfassend eine den Zeh untergreifende Auflagefläche sowie seitliche Anlagen für den Zeh,
   d a d u r c h   g e k e n n z e i c h n e t ,   daß die
   Richthilfe aus einem prinzipiell festen und formbeständigen, im Querschnitt im wesentlichen U-förmigen, sich
   in Richtung der Zehenachse erstreckenden schienenartigen Formstück (1) besteht, dessen U-Steg (2) die Auflagefläche und dessen U-Schenkel (3) die seitlichen Anlagen bilden.

2. Richthilfe nach Anspruch 1,   d a d u r c h
   g e k e n n z e i c h n e t ,   daß die U-Schenkel
   (3) über einen Teil ihrer Längserstreckung eine schwache
   Wölbung (31) nach außen aufweisen.

3. Richthilfe nach Anspruch 1 oder 2,   d a d u r c h
   g e k e n n z e i c h n e t ,   daß die U-Schenkel (3)
   an einem, dem hinteren Ende des Formstücks (1) mit
   gegenüber dem Ende der Auflagefläche (2) zurückspringenden Lappen (32) ausgebildet sind.

4. Richthilfe nach Anspruch 3,   d a d u r c h
   g e k e n n z e i c h n e t ,   daß die beiden Lappen
   (32) unterschiedlich lang sind.

5. Richthilfe nach Anspruch 3 oder 4,   d a d u r c h
   g e k e n n z e i c h n e t ,   daß einer der Lappen
   als längliche Lasche (33) ausgebildet ist.

6. Richthilfe nach einem der Ansprüche 1 bis 5,
   d a d u r c h   g e k e n n z e i c h n e t ,   daß
   an einem, dem vorderen Ende des Formstücks die Schenkelränder nach innen gebogen sind.

7. Richthilfe nach einem der Ansprüche 1 bis 6, d a d u r c h   g e k e n n z e i c h n e t ,   daß im vorderen Bereich der Auflagefläche (des U-Steges) des Formstücks eine plattenartige ebene Vertiefung (23) vorgesehen ist.

8. Richthilfe nach einem der Ansprüche 3 bis 7, d a d u r c h   g e k e n n z e i c h n e t ,   daß die Oberkanten der U-Schenkel (3) von den Lappen (32) her zum gegenüberliegenden, vorderen Ende leicht abfallen.

9. Richthilfe nach einem der Ansprüche 1 bis 8, d a d u r c h   g e k e n n z e i c h n e t ,   daß der U-Steg (2) mindestens im Bereich seines vorderen Abschlußrandes eine leichte Aufwölbung (21) aufweist.

10. Richthilfe nach einem der Ansprüche 1 bis 9, d a d u r c h   g e k e n n z e i c h n e t ,   daß zwischen den U-Schenkeln (3) eine sich im wesentlichen parallel zu dem U-Steg (2), nur über einen Teil der Länge des Formstücks (1) erstreckende bügelartige Verbindung ~~aufbringbar~~ *vorgesehen* ist.

11. Richthilfe nach einem der Ansprüche 1 bis 10, d a d u r c h   g e k e n n z e i c h n e t ,   daß sie aus einem metallischen Material wie Aluminium, rostfreiem Stahl od.dgl. besteht.

12. Richthilfe nach Anspruch 11,   d a d u r c h g e k e n n z e i c h n e t ,   daß sie mit einem durch Sintern oder Tauchen erzeugten Farbüberzug aus körperfreundlichem Material versehen ist.

13. Richthilfe nach einem der Ansprüche 1 bis 10, d a d u r c h   g e k e n n z e i c h n e t ,   daß

sie aus einem nichtmetallischen Material wie Kunststoff besteht.

14. Richthilfe nach einem der Ansprüche 1 bis 13,
dadurch gekennzeichnet, daß
das Formstück (1) an seinen Innenseiten mit Polsterungen versehen ist.

Fig. 1

Fig. 2

Fig. 3

**Europäisches Patentamt**

## EUROPÄISCHER RECHERCHENBERICHT

| | EINSCHLÄGIGE DOKUMENTE | | | EP 84730096.9 |
|---|---|---|---|---|
| **Kategorie** | **Kennzeichnung des Dokuments mit Angabe, soweit erforderlich, der maßgeblichen Teile** | | **Betrifft Anspruch** | **KLASSIFIKATION DER ANMELDUNG (Int. Cl. 4)** |
| A | DE - C - 505 040 (MANSFELD)<br><br>* Spalte 1, Zeilen 79-83; Fig. 1a *<br><br>-- | | 1 | A 61 F 5/14 |
| A | GB - A - 596 343 (HORNIK)<br><br>* Patentanspruch 1; Fig. 1,2 *<br><br>-- | | 1 | |
| A | US - A - 2 636 491 (BILLE)<br><br>* Patentanspruch 1; Fig. 1,2, 3 *<br><br>---- | | 1 | |
| | | | | **RECHERCHIERTE SACHGEBIETE (Int. Cl. 4)**<br><br>A 61 F |

Der vorliegende Recherchenbericht wurde für alle Patentansprüche erstellt.

| Recherchenort | Abschlußdatum der Recherche | Prüfer |
|---|---|---|
| WIEN | 11-03-1985 | MIHATSEK |

**KATEGORIE DER GENANNTEN DOKUMENTEN**
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82